# EUROPÄISCHE PATENTSCHRIFT

(11) **EP 0 727 231 B1**
(45) Veröffentlichungstag und Bekanntmachung des Hinweises auf die Patenterteilung: **19.09.2001**
(21) Anmeldenummer: 96101747.2
(22) Anmeldetag: 07.02.1996
(51) Int. Cl.: A61L 27/00

(54) **Verfahren zur Herstellung von Spongiosa-Knochenkeramikformkörpern**
Process for the preparation of spongiosa bone ceramic articles
Procédé pour la préparation d'articles en céramique osseuse spongieuse

(30) Priorität: 15.02.1995 DE 19504955
(43) Veröffentlichungstag der Anmeldung: 21.08.1996
(73) Patentinhaber: MERCK PATENT GmbH, 64293 Darmstadt (DE)
(72) Erfinder: Nies, Berthold, Dr., D-64407 Fränkisch-Crumbach (DE)

(56) Entgegenhaltungen:
- EP-A- 0 328 041
- US-A- 5 306 302
- DATABASE WPI Section Ch, Week 8740 Derwent Publications Ltd., London, GB; Class D22, AN 87-281769 XP002102646 -& JP 62 197066 A (AGENCY OF IND SCI & TECHNOLOGY), 31. August 1987 -& CHEMICAL ABSTRACTS, vol. 108, no. 22, 30. Mai 1988 Columbus, Ohio, US; abstract no. 192809, TAMATOSHI NOBUYUKI AND AL.: "Manufacture of biocompatible bone substitutes with porous block from silicon nitride and calcium phosphates" XP002102645

## Beschreibung

Die Erfindung betrifft ein Verfahren zur Herstellung von Spongiosa-Knochenkeramikformkörpem mit geometrisch unregelmäßigen Formen und abgerundeten Ecken und Kanten.

Es ist seit längerem bekannt, daß sich mineralisierter und zur Keramik gesinterter Knochen vorzüglich als Knochenersatzwerkstoff eignet. Bei Knochenkeramik liegt naturgemäß größte Übereinstimmung von chemischer Zusammensetzung, strukturellem Aufbau und mechanischer Festigkeit mit dem natürlichen Knochen vor. Spongiosa-Knochenkeramik erweist sich darüber hinaus als in besonderem Maße osteokonduktiv. Die offene, interkonnektierende, trabekuläre Struktur von spongiöser Knochenkeramik fördert das Auf- und Einwachsen neuer Knochenmatrix, so daß sich im Verlaufe der Einheilung eine intensive Durchbauung und damit Integration des Keramikimplantates ergibt. Knochenkeramiken hergestellt aus Knochen tierischer Herkunft werden daher vermehrt als Knochenersatzmaterialien bei der Osteosynthese und bei der Rekonstitution von krankheits- oder unfallbedingten Knochendefekten eingesetzt.

Die Herstellung von Knochenkeramik aus tierischem Knochen erfolgt in aller Regel in der Weise, daß man ausgewählte Knochen bzw. Knochenstücke zunächst mechanisch von allen anhaftenden Weichteilen befreit und durch Sägen zu Stücken geeigneter Form und Größe grob zurichtet, diese dann durch Entfernung aller weiteren organischen Anteile mineralisiert und schließlich die endgültige Formgebung vornimmt. Der Mineralisierungsvorgang beginnt zunächst mit mehrmaligem Auskochen in Wasser. Die weitere Behandlung kann etwa mit fett- bzw. eiweißlösenden Lösungsmitteln und/oder mit Hilfe von Wasserstoffperoxid erfolgen, wie beispielsweise in EP 0 141 004 beschrieben. Als besonders einfach und effektiv haben sich pyrolytische Mineralisierungsverfahren erwiesen wie zum Beispiel in US 5 306 302 beschrieben. Hierbei wird durch Hitzeeinwirkung der organische Anteil des Knochens zersetzt und der resultierende Kohlenstoff anschließend im Sauerstoffüberschuß ausgebrannt.

Für die Knochenpyrolyse sind Temperaturen zwischen 500 und 1000 °C üblich. Nach der Mineralisierung des Knochens erfolgt die Sinterung zur Keramik, wobei Temperaturen zwischen 800 und 1400 °C gebräuchlich sind. Durch die Sinterung erhält der Werkstoff erst die gewünschte endgültige Festigkeit. Bei den genannten Vorgängen ist besonders darauf zu achten, daß die poröse Struktur des ursprünglichen Knochens soweit als möglich erhalten bleibt. Bei der Umwandlung von spongiösem Knochenmaterial zu Spongiosa-Knochenkeramik kann vorzugsweise nach einem Verfahren gemäß DE 37 27 606 vorgegangen werden, in dem eine spezielle Führung von Temperatur und reduktivem bzw. oxidativem Charakter der Atmosphäre eine besonders schonende Pyrolyse ermöglicht.

Im weiteren Verlauf werden aus den rohen Spongiosa-Knochenkeramikstücken durch Sägen oder Fräsen Formkörper definierter Geometrie und Abmessung gefertigt. Handelsüblich sind beispielsweise Würfel, Quader und Zylinder mit Volumina von ca. 0,25 bis 4,25 cm³.

Die mögliche Ausbeute an derartigen Formkörpern, bezogen auf das ursprüngliche Rohmaterial, ist allerdings äußerst gering. Auf Grund der naturbedingten Form und Größe der Spongiosa-haltigen Knochenteile ist nur ein minderer Anteil des gesamten spongiösen Bereiches des Rohknochens zur Gewinnung der Formkörper geeignet und zugänglich. Corticalis, Wachstumsfugen, inhomogene und mechanisch instabile Bereiche müssen entfernt werden. Häufig sind solche Bereiche äußerlich nicht erkennbar, so daß die Formkörper spätestens beim Sägen bzw. Fräsen zu Bruch gehen oder nach diesem Vorgang als ungeeignet erkannt zu Ausschuß werden.

Die genannten geometrisch definierten Formkörper weisen allesamt scharfe Ecken und Kanten auf. In den Ecken- und Kantenbereichen ist die poröse Spongiosa-Keramik naturgemäß sehr empfindlich, so daß es zum Beispiel beim klinischen Einsatz sehr leicht zu Bruch und Abrieb kommt.

Neben ungewollter Formveränderung durch Bruch von Ecken und Kanten können abgetragene Partikel am Implantationsort unerwünschte Reaktionen hervorrufen. Diese Gefahr ist besonders bei der "Press-Fit"-Implantation derartiger Formkörper in entsprechend auf Maß gefräste Knochendefekte gegeben.

Die praktische Erfahrung im klinischen Einsatz von Spongiosa-Knochenkeramikformkörpern hat weiterhin gezeigt, daß ein zwingender Bedarf für vorgegebene Geometrien und Abmessungen nur in wenigen Fällen besteht. Häufig nimmt der Chirurg vor oder während der Implantation eine Bearbeitung der Formkörper vor, um sie in Form und Größe den aktuellen Gegebenheiten des Implantationsortes anzupassen. Hierbei läßt sich das unerwünschte Einbringen von feinen Partikel oft ebenfalls nicht vermeiden. Weiterhin wäre es in Fällen der Auffüllung großräumigerer Knochendefekte vielfach vorteilhafter eine größere Zahl von SpongiosaKnochenkeramikformkörpem mit geometrisch unregelmäßigen Formen und abgerundeten Ecken und Kanten an der Hand zu haben, um damit den Defekt zu verfüllen.

Es wurde nun gefunden, daß sich hierfür wünschenswerte SpongiosaKnochenkeramikformkörper mit geometrisch unregelmäßigen Formen und abgerundeten Ecken und Kanten sowie ohne mechanisch instabile Bereiche in der Weise erhalten lassen, daß man nach der Mineralisierung von in Stücken grob vorgeformten spongiösen Knochen und Sinterung zur Keramik die Formgebung durch Behandlung der Stücke in einer Kugelmühle vornimmt.

Gegenstand der Erfindung ist somit ein Verfahren zur Herstellung von Spongiosa-Knochenkeramikformkörpem, bei dem man in Stücken grob vorgeformten spongiösen Knochen durch Entfernung-aller organischen Anteile mineralisiert, dann die mineralisierte Knochenmatrix zur Keramik sintert und daran die endgültige Formgebung anschließt, das dadurch gekennzeichnet ist, daß die Formgebung der Knochenkeramikstücke durch Behandlung in einer Kugelmühle erfolgt, wobei mechanisch instabile Bereiche der Knochenkeramik abgetragen und geometrisch unregelmäßige Formkörper mit abgerundeten Ecken und Kanten erhalten werden.

Bei dem erfindungsgemäßen Verfahren wird zunächst in bekannter Weise, insbesondere nach dem Verfahren gemäß DE 37 27 606 vorgegangen, um das Knochenmaterial zu mineralisieren und zur Keramik zu sintem. Hierzu wird zunächst das ausgewählte Knochenmaterial, vorzugsweise Gelenkköpfe frisch geschlachteter Rinder, die durch Entfernen von Weichteilen und grobes Zurichten mittels Säge in quaderförmige Stücke vorbereitet sind, mehrmals in Wasser ausgekocht. Die Knochenstücke werden dann über mehrere Stunden hinweg getrocknet, unter Stickstoff pyrolisiert, unter Luft bzw. Sauerstoff ausgebrannt und dann der Endsinterung unterzogen. Vor der Endsinterung kann, wie in DE 40 28 683 beschrieben, ein Behandlungsschritt mit einer wäßrigen Lösung einer organischen Säure, insbesondere Zitronensäure, vorgesehen sein, durch den Calciumoxidanteile aus dem Material herausgelöst werden.

Erfindungsgemäß erfolgt die Endbehandlung der Knochenkeramikstücke zur Entfernung mechanisch instabiler Bereiche und zur eigentlichen Formgebung zu Formkörpem mit abgerundeten Ecken und Kanten in einer Kugelmühle. Hierzu können die Knochenkeramikstücke, gleich wie sie aus dem Sinterprozeß anfallen, herangezogen werden. Zweckmäßig ist eine Vorzerkleinerung mittels Säge, Fräse oder Backenbrecher, die sich einerseits an der Entfernung etwaiger restlicher Corticalis-Bereiche und Wachstumgsfugen und an der gewünschten Größenordnung der Formkörper orientiert. Gleichermaßen geeignet sind auch Bruch und Abschnitte, die aus der Produktion von Formkörpern definierter Geometrie anfallen.

Die grob vorgeformten Spongiosa-Knochenkeramikstücke werden nun in eine der jeweiligen Kapazität entsprechenden Menge in eine handelsübliche Kugelmühle verbracht und dort durch mehr oder weniger intensive Behandlung der endgültigen Formgebung unterzogen. Eine typische Kugelmühle ist im wesentlichen aufgebaut aus einem zylindrischen Gefäß aus einem ausreichend harten Werkstoff, das mit einem Deckel dicht verschlossen werden kann. Dieses Gefäß wird auf einem Walzenstuhl um seine Achse gedreht mit zweckmäßigerweise regulierbarer Drehgeschwindigkeit. Alternative Bauformen sind Fliehkraft-, Schwing- und Planeten-Kugelmühlen. Derartige Kugelmühlen haben typischerweise ein Volumen zwischen 1 und 15 I.

Üblicherweise werden dem Mahlgut während der Behandlung Mahlkörper aus einem ausreichend harten Material, in der Regel in Kugelform, zugegeben. Die Mahlkugeln haben typischerweise Durchmesser zwischen 9 und 50 mm. Zweckmäßigerweise bestehen Kugelmühle und Mahlkugeln aus Hartporzellan oder Al₂O₃. Letzteres Material besitzt gegenüber Knochenkeramik eine deutlich höhere Härte, so daß nicht mit Verschleiß der Mühle und Kontamination des Mahlgutes zu rechnen ist.

Intensität und Dauer der Bearbeitung in der Kugelmühle sowie etwaige Zugabe und Größe der Mahlkugeln sind abhängig von Primärgröße und Qualität der Knochenkeramikstücke, dem gewünschten Abtrag und der angestrebten Endgröße. Ein langsamer Lauf der Mühle über längere Zeit hinweg ohne oder nur mit kleinen Mahlkugeln ist schonender und ist etwa bei Spongiosa-Keramik geringerer Dichte und Festigkeit vorzuziehen. Eine kurze intensive Behandlung mit relativ großen Kugeln kann bei dichterem, hartem Material zweckmäßig sein. Typische Mahlbedingungen sind zum Beispiel eine Laufzeit von etwa 2 bis 6 Stunden mit Mahlkörpern bzw. eine Laufzeit von etwa 6 bis 18 Stunden ohne Mahlkörper in einer Kugelmühle von 10 l Inhalt mit einer Füllung von ca. 5 l Schüttvolumen an Mahlgut.

Neben der "trockenen" Behandlung in der Kugelmühle kann es zweckmäßig sein, die Formgebung "feucht" durchzuführen. Hierzu gibt man dem Mahlgut zusätzlich eine Mahlhilfsflüssigkeit zu, wobei das Volumenverhältnis Mahlgut zu Flüssigkeit im Bereich 0,5 bis 2,5 liegen kann. Die Behandlung kann hierdurch noch schonender erfolgen. Außerdem werden die abgetragenen Partikel zu einem großen Teil von den behandelten Knochenkeramikstücken abgelöst bzw. abgeschwemmt. Als Flüssigkeit wird vorzugsweise Wasser eingesetzt. Es können auch organische Lösungsmittel als Mahlhilfsflüssigkeit verwendet werden. Vorzugsweise kommen Alkohole wie insbesondere Methanol und Ethanol in Frage. Es kann auch sinnvoll sein, eine wäßrige Salzlösung, vorzugsweise von Calciumsalzen oder Phosphatsalzen als Mahlhilfsflüssigkeit einzusetzen. Hierdurch lassen sich gewünschtenfalls bereits während des Mahlvorganges die Knochenkeramikformkörper chemisch modifizieren, etwa durch eine zusätzliche Belegung der Oberfläche mit Calcium-lonen, wodurch dieEinhei lungeines derartigen Implantationskörpers positiv beeinflußt werden kann.

Das Ergebnis der erfindungsgemäßen Behandlung in der Kugelmühle ist eine vollständige Entfernung der mechanisch gering oder nicht belastbaren Anteile der grob vorgeformten Knochenkeramikkörper. Die Körper werden je nach Art und Dauer der Behandlung mehr oder weniger stark abgerundet. Insbesondere die schwachen Stellen an den exponierten Ecken und Kanten werden entfernt und können beim späteren klinischen Einsatz nicht mehr wegbrechen und/oder Partikel freisetzen. Nach der Behandlung in der Kugelmühle bleiben etwa 60 bis 80 % des eingesetzten Knochenkeramikmaterials als abgerundete unregelmäßig geformte Körper übrig. Diese unregelmäßigen Körper weisen im Vergleich zum eingesetzten Material eine höhere Homogenität, bezogen auf die Porosität, auf. Als Formkörper verbleiben aufgrund der Bearbeitung lediglich die spongiösen Bereiche höherer Dichte und mechanischer Stabilität, da alle hochporösen und mechanisch instabilerBereiche abgetragen worden sind.

Die erfindungsgemäße Behandlung wird durch Aussieben und anschließend durch eine gründliche Auswaschung des Abriebes abgeschlossen. Nach ausreichender Waschung erzeugen die erfindungsgemäß behandelten Körper weder bei der Lagerung noch bei der klinischen Handhabung nennenswerten Abrieb. Durch die zuverlässige Entfernung aller gering belastbaren überdurchschnittlich hochporösen Spongiosa-Regionen verbleibt ein Material von deutlich höherer Druckfestigkeit, was für den klinischen Einsatz essentiell ist. Die erheblich bessere Ausnutzung des eingesetzten Knochenmaterials ist ein weiterer wesentlicher Vorteil des erfindungsgemäßen Verfahrens, der die Wirtschaftlichkeit des Einsatzes von Spongiosa-Knochenkeramik in der Endoprothetik steigert

### Beispiel 1

Rohe, von Weichteilen befreite Gelenkköpfe frisch geschlachteter Rinder werden mittels Säge in quaderförmige Stücke der Abmessungen von grob 30 x 30 x 100 mm zerteilt und diese dreimal ca. 1 Std. lang mit Wasser ausgekocht.

Die Knochenstücke werden dann 6 Std. lang bei 110 °C getrocknet Danach werden diese unter Stickstoffatmosphäre innerhalb von 9 Std. auf 450 °C erhitzt. Während einer anschließenden 20stündigen Aufheiz-periode von 450 °C auf 600 °C wird die Atmosphäre linear auf Luftsauerstoff umgestellt und danach innerhalb von 5 Std. auf 900 °C erhitzt. Nach dem Abkühlen werden die Stücke in ein Bad von 5 Gew. %iger Zitronensäurelösung (10 l pro 1 kg Knochenmaterial) verbracht und in dem bewegten Bad bei einer Temperatur von 20 °C für einen Zeitraum von 3 Std. behandelt. Nach dieser Behandlung werden die Stücke 3 mal mit demineralisiertem Wasser nachgewaschen.

Zur Endsinterung werden die Stücke innerhalb von 21 Std. auf 1250 °C erhitzt, 3 Std. auf dieser Temperatur gehalten und dann frei abgekühlt.

Die erhaltenen Knochenkeramikstücke zeigen die unveränderte poröse Struktur des ursprünglichen spongiösen Knochens. Röntgenographisch besteht die Keramik zu etwa 99 % aus Hydroxylapatit.

Aus den so erhaltenen Knochenkeramikstücken werden mit Hilfe einer Hohlfräse mit 10 mm Innendurchmesser Zylinder gefräst, die dann auf 10 mm Länge gebracht werden. Bei diesem Vorgang braucht nicht auf Homogenität, Wachstumsfugen, Risse usw. geachtet werden, wie es bei der Herstellung von geometrisch definierten Formkörpern notwendig ist.

Die Menge derartiger Zylinder, die einem Schüttvolumen von ungefähr 5 I entspricht, wird in eine handelsübliche Kugelmühle aus Al₂O₃ mit einem Fassungsvermögen von 10 l gegeben und 18 Stunden ohne Zusatz von Mahlkörpern mit 30 Umdrehungen pro Minute bearbeitet.

Anschließend werden die Formkörper dreimal mit demineralisiertem Wasser im Ultraschallbad gewaschen und dabei mehrfach aufgeschüttelt. Die Formkörper werden 15 Minuten bei 122 °C im strömenden Dampf autoklaviert. Danach wird das Material getrocknet und 18 Stunden lang bei 300 °C unter Luftzutritt von organischen Kontaminationen befreit. Man erhält gut abgerundete bis angenähert kugelförmige Formkörper von hoher Homogenität und mechanischer Festigkeit, welche zu jeweils fünf Stück in Tiefziehverpackungen eingesiegelt werden.

### Beispiel 2

Es wird wie in Beispiel 1 vorgegangen, jedoch wird hier Abfallmaterial, Bruch und Ausschuß aus der Herstellung geometrisch definierter Formkörper benutzt. Man erhält Körper unterschiedlicher Form und Größe mit gut abgerundeten Ecken und Kanten und von hoher Homogenität und mechanischer Festigkeit.

### Beispiel 3

Es wird wie in Beispiel 1 vorgegangen, allerdings wird hier das gesinterte Material nach Entfernung von Corticalis-Bereichen im Backenbrecher auf Stücke von 1 bis 2 cm Größe zerkleinert und dann unter Zusatz von 5 Al₂O₃-Kugeln mit 3 cm Durchmesser 3 Stunden lang in der Kugelmühle behandelt. Hierbei werden Stücke von hoher Dichte und variabler Form erhalten, die zur Sichtung über ein Sieb gegeben werden. Nach Entfernung des Abriebs im Ultraschallbad und anschließender Autoklavierung und Trocknung werden die Stücke zu Einheiten von jeweils 20 g abgepackt.

## Patentansprüche

1. Verfahren zur Herstellung von Spongiosa-Knochenkeramikformkörpern, bei dem man in Stücken grob vorgeformten spongiösen Knochen durch Entfernung aller organischen Anteile mineralisiert, dann die mineralisierte Knochenmatrix zur Keramik sintert und daran die endgültige Formgebung anschließt, **dadurch gekennzeichnet, daß** die Formgebung der Knochenkeramikstücke durch Behandlung in einer Kugelmühle erfolgt, wobei mechanisch instabile Bereiche der Knochenkeramik abgetragen und geometrisch unregelmäßige Formkörper mit abgerundeten Ecken und Kanten erhalten werden.

2. Verfahren nach Anspruch 1, **dadurch gekennzeichnet, daß** die Formgebung in einer Kugelmühle aus Al₂O₃ unter Zugabe von Kugeln aus Al₂O₃ erfolgt.

3. Verfahren nach den Ansprüchen 1 oder 2, **dadurch gekennzeichnet, daß** die Formgebung in Anwesenheit von Wasser, einer wäßrigen Salzlösung oder von einem organischen Lösungsmittel erfolgt.

## Claims

1. Process for the production of shaped spongiose bone ceramic articles in the course of which spongiose bones which have been roughly preshaped into pieces are mineralized by the removal of all organic components, the mineralized bone matrix is then sintered to form the ceramic, and then the final shaping operation is carried out, **characterized in that** the operation of shaping the bone ceramic pieces is carried out by treatment in a ball mill, mechanically unstable regions of the bone ceramic being abraded to give geometrically irregular articles with rounded corners and edges.

2. Process according to Claim 1, **characterized in that** the shaping operation is carried out in a ball mill made of Al₂O₃ with the addition of balls made of Al₂O₃.

3. Process according to Claim 1 or 2, **characterized in that** the shaping operation is carried out in the presence of water, an aqueous salt solution or an organic solvent.

## Revendications

1. Procédé pour la préparation d'articles en céramique osseuse spongieuse, par lequel on minéralise des os spongieux grossiers préformés en morceaux grâce à l'extraction de toutes les parties organiques, par lequel on fritte ensuite la matrice osseuse minéralisée, et à laquelle est affilié le faconnage final, **caractérisé en ce que** le faconnage des morceaux de céramique osseuse s'effectue par un traitement dans un broyeur à billes, moyennant quoi des zones mécaniquement instables de la céramique osseuse sont retirees et des articles géométriquement irréguliers présentant des coins et des angles arrondis sont conservés.

2. Procéde selon la revendication 1, **caractérisé en ce que** le faconnage s'effectue dans un broyeur à billes composé de Al₂O₃ en ajoutant des billes composées de Al₂O₃.

3. Prodéde selon la revendication 1 ou 2, dans lequel le faconnage s'effectue en présence d'eau, d'une solution saline aqueuse ou d'un solvant organique.
